# EUROPEAN PATENT SPECIFICATION

(11) **EP 1 411 035 B1**
(45) Date of publication and mention of the grant of the patent: **09.12.2009**
(21) Application number: 03023327.4
(22) Date of filing: 15.10.2003
(51) Int. Cl.: C04B 38/10, C04B 35/447, A61L 27/12, A61L 27/56

(54) **A process for the production of porous calcium phosphate articles for bone regeneration and as a support for cells and the porous articles themselves**
Verfahren zur Herstellung porösen Calciumphospat Körpern zur Knochenregeneration und wie Zellenträger, und die erhaltene poröse Körper
Procédé de fabrication d'articles poreux à base de phosphate de calcium pour la régénération osseuse et comme support pour cellules, et les articles poreux obtenus

(30) Priority: 15.10.2002 IT BO20020650
(43) Date of publication of application: 21.04.2004
(73) Proprietor: FIN - CERAMICA FAENZA S.P.A., 48018 Faenza (IT)
(72) Inventor: Belpassi, Andrea, 61029 Urbino (IT); Dolcini, Laura, 48018 Faenza (Ravenna) (IT); Martinetti, Roberta, 47100 Forli (IT)
(74) Representative: Bottero, Carlo

(56) References cited:
- US-A1- 2002 114 938
- US-B1- 6 340 648

## Description

The present invention relates to a process for the production of porous calcium phosphate articles for bone regeneration and as a support for cells and the porous articles themselves.

More specifically, the present invention relates to the technology for production of calcium phosphate based articles characterised by a total porosity in the range between 70 and 90% by volume for use in the biomedical sector, in particular for bone regeneration and as support designed to hold cells.

Calcium phosphates and in particular ceramic hydroxyapatite (also known to experts in the field as HA) were designed for use as bone fillers many years ago and their behaviour from the biocompatibility viewpoint is well known.

Ceramic hydroxyapatite appears to be the ideal material to substitute bone, in tissue regeneration and engineering. Ceramic hydroxyapatite is known as an osteoconductive material. This means that it can conduct bone formation. Bone formation occurs directly on the surface of the material, creating a strong bond with the bone tissue.

The intrinsic characteristics of the material have an effect on the biological result of an in vivo graft, including the properties of the powder, the properties of the sintered material and its structural configuration. In other words, the biological properties of a product do not depend solely on its chemical composition, but also on the form, understood to be the structure of the material: dimensions and shapes of the pores.

The properties of porous hydroxyapatite make it more integrable, "absorbable" and more osteoconductive than dense hydroxyapatite. When designing a bone substitute it is important to consider all of these parameters. In particular, as described above, porosity is essential in order to obtain more rapid osteoconduction.

Since both medical knowledge and surgical techniques are constantly developing, there is a continuous growth in the demand for bone substitutes of synthetic origin in orthopaedic surgery, neurosurgery and maxillofacial surgery.

Patent EP-0 598 783 proposes a process for obtaining porous articles starting with a dispersion of particles of refractory material in a liquid base to form a low-viscosity composition which is made porous by agitation in a free atmosphere or with other methods including: bubbling through a sponge, pressurised gases, reactive gas generating substances. The dispersion may include surfactants and polymerisable monomers.

However control of the porosity of the articles obtained with the process described in the above-mentioned patent is uncertain, meaning that the required porosity and pore size distribution cannot always be obtained.

US 6,340,648 B1 discloses a calcium phosphate porous sintered body having a porous structure having the features as set forth in col. 2, lines 40-48. The method for producing the above porous sintered body comprises the steps of: preparing a slurry by dispersing and/or dissolving a calcium phosphate powder and an organic compound, hardenable by cross-linking polymerization, in a solvent; foaming a slurry to prescribed volume by stirring and/or gas introduction with addition of foaming agent to the slurry; foaming a compact by slip-cutting after adding of cross-linking agent and/or initiator to the slurry for hardening it by cross-linking of organic compact and drying the compact followed by sintering. A dispersant, a bubble-shaping agent, a thicker or the like may be added to the slurry. As more clearly reported in Example 1, the foaming of the slurry is simply obtained "by mechanical stirring".
US 2002/0114938 A1 relates to a porous sintered body of a calcium phophate-based ceramic and to a method for producing it. The above method comprises the steps of: (1) preparing a slurry comprising a calcium phosphate-based ceramic powder, a watersoluble high molecular compound and a nonionic surface active agent; (2) stirring the slurry vigorously to froth the slurry; (3) solidifying the frothed slurry into a gel; and (4) drying and sintering the gel. Moreover, at para [0012] it is disclosed that the slurry is stirred while passing a gas though the slurry to froth the slurry.

One aim of the present invention is to provide an improved process for the production of porous calcium phosphate articles with a porous structure having a porosity controlled both as a ratio of the volume of material to the volume of air and as the porosity and pore size distribution.

Another aim of the present invention is to produce a porous calcium phosphate article with an improved porous structure, able to promote in particular both bone generation and holding cells by acting as a support. Use of this porous article avoids all risks of viral infections due to the potential risks associated with use of materials of human or animal origin.

Accordingly, the present invention presents a process for the production of porous calcium phosphate articles with a porous structure as specified in claim 1.

The dependent claims refer to preferred, advantageous embodiments of the invention.

Preferred embodiments of the present invention are illustrated in the accompanying drawings, without limiting the scope of the inventive concept, and in which:
- Figure 1 illustrates the appearance of the material with fine texture porosity of a porous article made according to the present invention;
- Figure 2 illustrates the appearance of the material with medium texture porosity of a porous article made according to the present invention;
- Figure 3 illustrates the appearance of the material with large texture porosity of a porous article made according to the present invention;
- Figure 4 illustrates the appearance of part of the structure of a porous article made according to the present invention;
- Figure 5 illustrates the appearance of the microstructure of a porous article made according to the present invention;
- Figure 6 illustrates the structure of material with fine texture porosity of a porous article made according to the present invention;
- Figure 7 illustrates the structure of material with medium texture porosity of a porous article made according to the present invention;
- Figure 8 illustrates the structure of material with large texture porosity of a porous article made according to the present invention;
- Figure 9 illustrates an apparatus for the production of material for a porous article made according to the present invention;
- Figure 10 is a flow chart illustrating the steps in the process according to the present invention.

The process for the production of a porous article disclosed involves a step of preparing a ceramic mixture, known to experts as a "slip" and hereinafter referred to with this term, using calcium phosphate powder and a deflocculating agent, mixed with a liquid.

When preparing the slip, the consistency must be quite viscous, although it must still be possible to cast the slip in a mould. For this reason, the components used to make the slip must be added gradually and while constantly mixing.

When preparing the ceramic slip, the viscosity obtained must preferably be in the range between 10,000 mPa.s and 40,000 mPa.s, and more preferably in the range between 25,000 mPa.s and 28,000 mPa.s (considering γ = 4 s ⁻¹ where γ = rate of deformation to which the slip is subjected).

The ceramic powder used in the slip has an average particle size of between 0.7 and 2.0 µm, preferably 1.4 µm with distribution of the size of the particles from 0.2 to 60 µm, most preferably with distribution of the size of the particles (from the smallest particle to the largest) in the range between 0.5 and 10 µm.

The ceramic powder is also characterised by a specific surface area value between 2 and 8 m²/g, preferably between 4 and 6 m²/g according to the B.E.T. method which, as is known, is an analysis method based on physical and chemical adsorption of a gas on a solid.

The ceramic powders considered in the present invention are monophasic powders such as:
hydroxyapatite (HA), Beta-Tricalcium Phosphate (β-TCP), Alpha-Tricalcium Phosphate (α-TCP);
biphasic powders such as:
   hydroxyapatite / Beta-Tricalcium Phosphate (HA / β-TCP), hydroxyapatite / Alpha-Tricalcium Phosphate (HA/α-TCP) and Beta-Tricalcium Phosphate / Alpha-Tricalcium Phosphate (β-TCP/ α-TCP); and
   other calcium/phosphate powders containing compounds and/or elements such as CO₃ and Mg, carbonated hydroxyapatite (CHA) and hydroxyapatite with magnesium (HA-Mg).

The liquid base of the slip is double-distilled water and accounts for between 19% and 25% of the weight of the slip, preferably between 21% and 23% by weight.

A deflocculating agent is used to obtain a stable slip: for example, products in the chemical class of alkali-free polyelectrolytes.

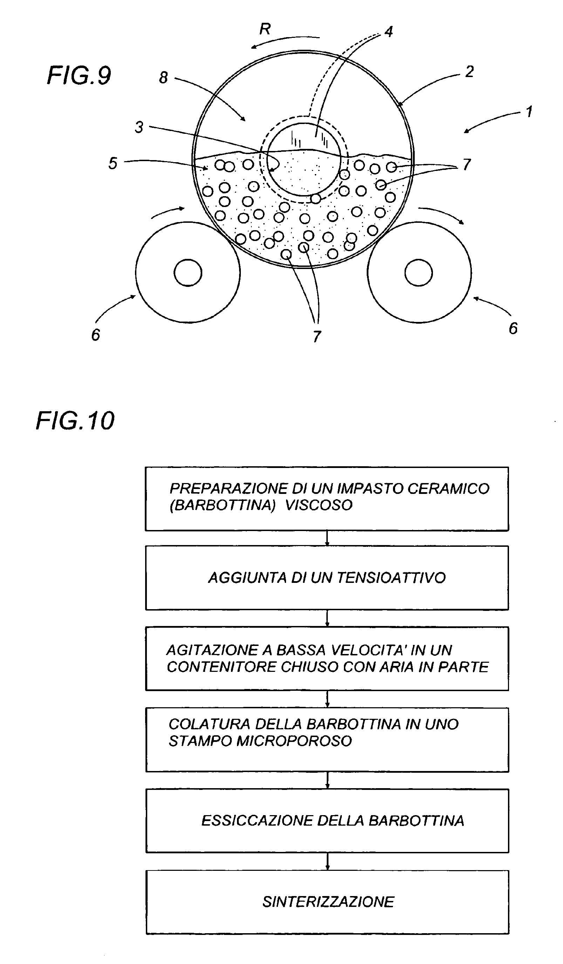

Figure 9 illustrates an example of a mixer 1 for obtaining the slip. The mixer comprises a closable container 2, substantially in the form of a bottle positioned with the axis horizontal and having an opening 3 with a cap 4 that closes it. The opening 3 can be used to introduce the ingredients of the slip 5. The ingredients are mixed by rotary means 6, consisting of rotary rollers with horizontal axes which cause the container 2 to rotate by friction.

The homogeneity of the slip is obtained by mixing the raw materials with the aid of grinding means 7, for example consisting of alumina grinding balls.

All hard and chemically inert materials can be used for the grinding means 7, so as to promote homogenisation of the raw materials and not create reactions with the raw materials.

In particular, alumina grinding means may be used both because they have chemically inert behaviour and because of their hardness: this gives them a high level of grinding power.

The preferred quantity of grinding means used relative to the quantity of slip has a ratio of between 0.75:1 and 1.5:1, and preferably 1:1. The mixing time is preferably between 5 and 10 hours with low speed rotation of the mixing means 6. For example, the container 2 may rotate at 10 - 20 revolutions per minute, preferably 12 - 18 rpm; even more preferably at 15 rpm.

The process then involves the step of slip expansion, modifying its surface activity by adding one or more anionic surfactants selected from the group of alkyl sulphates, sodium salts and ammonium salts.

Therefore, the various porosities are obtained by changing the surface activity of the slip by adding to the slip one and/or more anionic surfactants, for example: DACPO/27, COSMACOL/AES, Schäumungsmittel W53 FL and Olimpicon A - in quantities of between 0.5 and 4% by weight, and preferably between 1 and 3 % by weight.

Following the addition of surfactants to the slip, homogenisation of the mixture is achieved by rotating the container 2 for a time preferably between 2 and 20 hours (preferably 5 - 10 hours) at a low container 2 speed of rotation. For example, container 2 rotation may be between 10 and 20 rpm, preferably 12 - 18 rpm.

It should be noticed that homogenisation must occur in a closed environment, that is to say, in the closed container 2 in which there is a predetermined volume 8 of air or inert gas. Since the volume 8 is fixed and predetermined, the exact quantity of air or inert gas incorporated in the slip is guaranteed.

The homogenisation time, the speed of rotation of the container 2, the percentage and/or type of surfactant allow complete homogenisation of the slip with all of the air or inert gas present in the closed container 2.

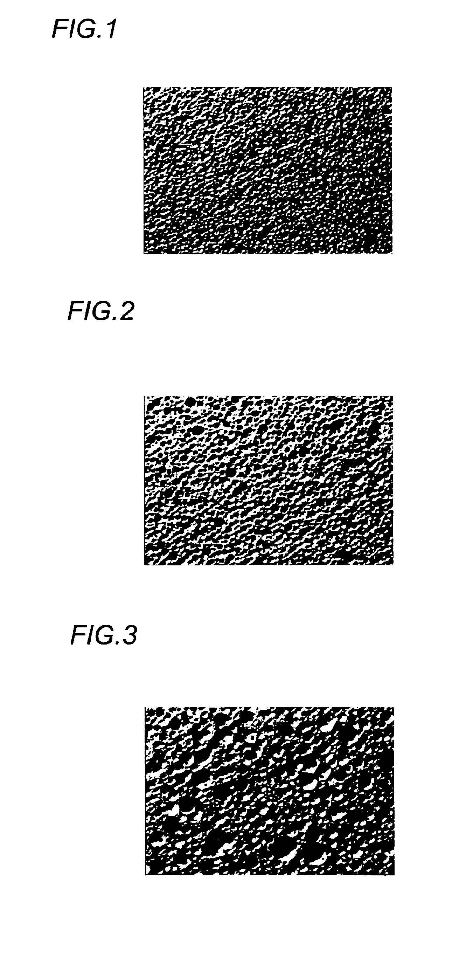

With reference to the type of porosity to be obtained - fine texture, medium texture and large texture (Figures 1, 2 and 3) different percentages and/or types of anionic surfactants are added to the basic slip.

The difference in terms of total porosity (from 70 to 90% by volume) may be planned with the present invention. It is closely related to the reaction volume of the closed container used during the step of homogenising the slip with the surfactant(s). The reaction volume of the air or inert gas is between 35% and 70% of the total volume of the container 2, preferably being between 40% and 60%, so as to obtain total porosity in a range of between 70% and 90% by volume.

The total percentage porosity of the final device depends on the volume of air made available to the expansion reaction of the slip with surfactant added to it: the greater the volume of air made available to the reaction, the greater the total porosity of the device.

This is followed by the step of casting the slip thus obtained in a porous mould.

The mould is preferably made of porous paper (weight of paper 150g/m²). The porosity of the mould is preferably in a range of 15 - 30 µm, and preferably in a range of 17 - 25 µm. This is followed by a step of drying the slip cast in the porous mould. Drying may take place at room temperature (keeping the temperature in the range 20 - 25 degrees Centigrade), or drying may take place in a microwave oven at the controlled temperature of 25 degrees Centigrade.

When drying at room temperature, the time is in a range of 18 - 50 hours, most preferably 24 - 48 hours, whilst when using a microwave oven, keeping the temperature constant at 25 degrees Centigrade, the drying time is lowered to a range of between 5 and 24 hours, most preferably 8 - 20 hours.

When drying in a microwave oven, the slip used to make the porous article is dried evenly at all points. This avoids the problems which could arise with conventional stoves, caused by different expansion between the colder inside and the hotter outside of the material and the porosity of the slip.

This is followed by a step of sintering the article to consolidate the structure obtained. Possible temperatures for sintering are in the range between 400 and 1400 degrees Centigrade, most preferably in the range between 900 and 1300, with a preferred value of approximately 1200 degrees Centigrade.

The sintering step may also be carried out in a flow of carbon dioxide (CO₂), in particular for carbonated hydroxyapatite and to prevent the carbon present in the hydroxyapatite from combining with oxygen.

After sintering, the porous article can easily be processed to obtain the shapes necessary in the various medical sectors. The porous article obtained using the present invention may be used as a bone substitute for filling bone defects or as a support for cells in hard tissue engineering.

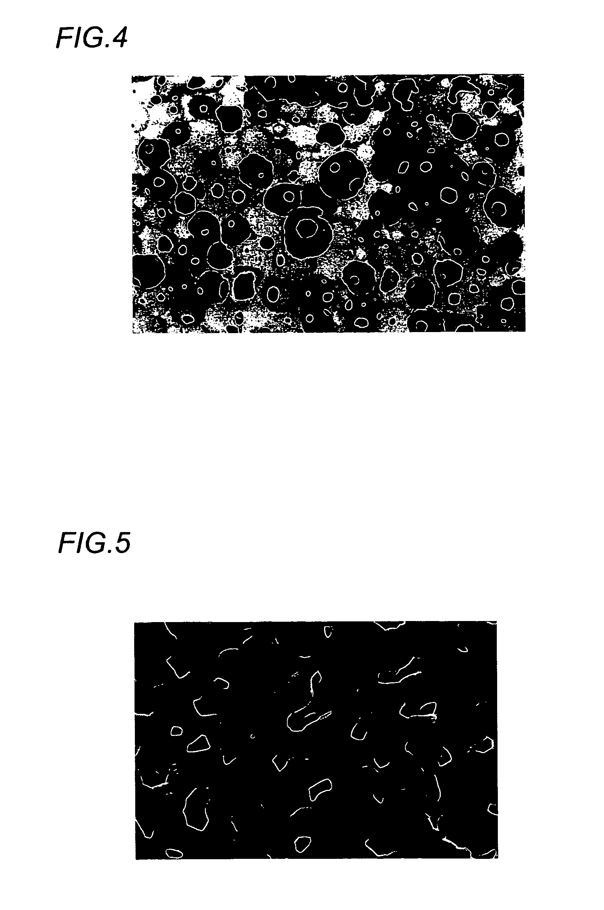

The macroporosity of the sintered porous article obtained as described using the present invention is characterised by an open porosity (Figure 4).

The microstructure of the articles produced in accordance with the present invention is illustrated in Figure 5. It may be controlled by modifying the final temperature reached during the consolidation process (sintering). The porous articles obtained using the process disclosed have excellent even porosity and pore size distribution.

The total distribution of the porosity (micro, macro and interconnecting pores) was analysed using an image analyser (Leica Imaging System Ltd. Q500MW by Qphase Application).

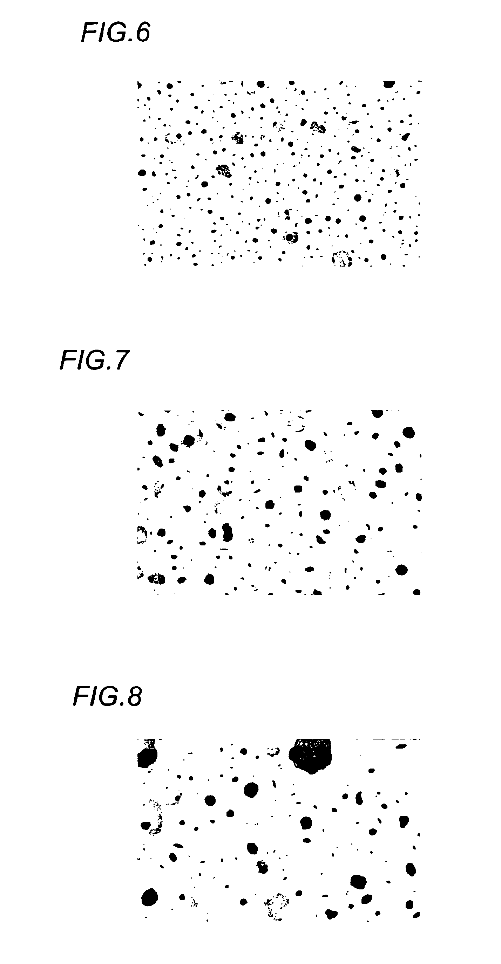

The data about pore size distribution relative to the various types of texture is indicated in Table 1, whilst in Table 2, in particular, the data refers to the interconnecting porosity distribution (Figures 6, 7 and 8).

The porosity data is expressed in terms of equivalent diameter. Equivalent diameter is understood to mean the average value calculated considering the smallest diameter and the largest diameter of the pore.

**Table 1 - Total porosity distribution**

| Ref. Sample | 0 -100 µm Area*(%) | 100-200µm Area*(%) | 200-500 µm Area*(%) | >500 µm Area*(%) |
|---|---|---|---|---|
| HA (Fine Texture) | 4.48 | 35.34 | 54.94 | 5.24 |
| HA (Medium Texture) | 1.10 | 22.35 | 58.28 | 18.27 |

| | | | | |
|---|---|---|---|---|
| * surface area analysed = 350 mm² | | | | |

**Table 2 - Interconnecting porosity distribution**

| Ref. Sample | < 80 µm Area*(%) | 80 -120 µm Area*(%) | 120 - 200µm Area*(%) | > 200µm Area*(%) |
|---|---|---|---|---|
| HA (Fine Texture) | 81.78 | 16.01 | 2.21 | 0 |
| HA (Medium Texture) | 54.72 | 25.79 | 16.05 | 3.44 |
| HA (Large Texture) | 47.03 | 19.76 | 15.42 | 17.79 |

| | | | | |
|---|---|---|---|---|
| * surface area analysed = 60 mm² | | | | |

The following are several examples of the process disclosed. These examples must only be considered for improved understanding and in no way limit the scope of the present invention.

### EXAMPLE 1

Hydroxyapatite powder, water and Dolapix CA (manufacturer: ZSCHIMMER & SCHWARZ) are mixed with the aid of alumina grinding means to obtain a slip (ratio of slip/grinding means = 1:1.5).

The following surfactants: SCHÄUMUNGSMITTEL W53 FL (trade name, manufacturer: ZSCHIMMER & SCHWARZ), DACPON/27-23 AL (manufacturer: Condea*)* are added to the slip in a closed container with a reaction volume, that is to say, the volume of air inside the container, in the range between 60 and 65% of the total volume.

Following addition of the surfactants, the slip is mixed by rotating the closed container for 10 hours at a speed of 15 rpm. After the slip has been mixed, it is cast in a porous mould then the product is dried for 24 hours at room temperature.

At the end of the drying process the green sample is sintered for 1 hour at a temperature of 1200 degrees Centigrade.

The sample obtained has a structure with open porosity characterised by a total porosity which is 90% of the volume (±3%) with large texture (Figures 3 and 8).

### EXAMPLE 2

Hydroxyapatite powder, water and Dolapix (manufacturer: ZSCHIMMER & SCHWARZ) are mixed with the aid of alumina grinding means to obtain a slip (ratio of slip/grinding means = 1:1). The following surfactants are added to the slip prepared in this way: SCHÄUMUNGSMITTEL W53 FLUSSIG (manufacturer: ZSCHIMMER & SCHWARZ), DACPON/27-23 AL (manufacturer: Condea), COSMACOL AES 70-2-25 NE (manufacturer: Condea) in a closed container with a reaction volume, that is to say, the volume of air inside the container, in the range between 50 and 55% of the total volume.

Following addition of the surfactants, the slip is mixed by rotating the closed container for 12 hours at a speed of 15 rpm. After the slip has been mixed, it is cast in a porous mould then dried for 36 hours at room temperature. At the end of the drying step the green sample is sintered for 1 hour at a temperature of 1200 degrees Centigrade. The article obtained has a structure with open porosity characterised by a total porosity which is 80% of the volume (±3%) with medium texture (Figures 2 and 7).

### EXAMPLE 3

Hydroxyapatite powder, water and Dolapix CA (manufacturer: ZSCHIMMER & SCHWARZ) are mixed with the aid of alumina grinding means to obtain a homogeneous slip (ratio of slip/grinding means = 1:0.75). The following surfactant is added to the slip prepared in this way: SCHÄUMUNGSMITTEL W53 FL (manufacturer: ZSCHIMMER & SCHWARZ) in a closed container with a reaction volume (volume of air inside the container) in the range between 40 and 45% of the total volume. Following addition of the surfactant, all of the ingredients are mixed by rotating the closed container for 18 hours at a speed of 15 rpm. After the slip has been mixed, it is cast in a porous mould then dried for 40 hours at room temperature.

At the end of the drying process the green article is sintered for 1 hour at a temperature of 1200 degrees Centigrade.

The end article has a structure with open porosity, with a total porosity which is 70% of the volume (±3%), characterised by a fine texture (Figures 1 and 6).

### EXAMPLE 4

Hydroxyapatite powder, water and Dolapix CA (manufacturer: ZSCHIMMER & SCHWARZ) are mixed with the aid of alumina grinding means to obtain a homogeneous slip (ratio of slip/grinding means = 1:1). The following surfactants are added to the slip prepared in this way: SCHÄUMUNGSMITTEL W53 FL (manufacturer: ZSCHIMMER & SCHWARZ) and OLIMPICON A (manufacturer: Olimpia) in a closed container with a reaction volume (volume of air) in the range between 50 and 55% of the total volume.

Following addition of the surfactants, all of the ingredients are mixed by rotating the closed container for 5 hours at a speed of 15 rpm. After the slip has been mixed, it is cast in a porous mould then dried for 48 hours at room temperature.

At the end of the drying process the green article is sintered for 1 hour at a temperature of 1200 degrees Centigrade.

The end article has a structure with open porosity, with a total porosity which is 80% of the volume (±3%), characterised by a medium texture (Figures 2 and 7).

### EXAMPLE 5

Carbonated hydroxyapatite powder (CHA), water and Dolapix CA (manufacturer: ZSCHIMMER & SCHWARZ) are mixed with the aid of alumina grinding means to obtain a homogeneous slip (ratio of slip/grinding means = 1:1). The following surfactants are added to the slip prepared in this way: SCHÄUMUNGSMITTEL W53 FL (manufacturer: ZSCHIMMER & SCHWARZ) and OLIMPICON A (manufacturer: Olimpia) in a closed container with a reaction volume (volume of air) in the range between 55 and 60% of the total volume.

Following addition of the surfactants, all of the ingredients are mixed by rotating the closed container for between 1 and 2 hours at a speed of 15 rpm. After the slip has been mixed, it is cast in a porous mould then dried for 48 hours at room temperature.

At the end of the drying process the green article is sintered for 1 hour at a temperature of 600 degrees Centigrade, in a flow of CO₂.

The end article has a structure with open porosity, with a total porosity which is 83% of the volume (±3%), characterised by a medium-large texture (a texture whose appearance is between those in Figures 2, 7 and 3, 8 ) .

The present invention brings important advantages.

The process disclosed may be used to produce porous articles whose porosity is controlled both in terms of the overall porosity volume and in terms of porosity distribution.

Controlling the porosity is simple, since it depends on few parameters: in particular, the volume of air (or inert gas) in the closed container 2 allows the exact quantity of air or inert gas which is incorporated in the slip to be obtained.

As regards the type of porosity to be obtained: fine texture, medium texture and large texture (Figures 1, 2 and 3), these are obtained by adding different percentages and/or types of anionic surfactants to the basic slip.

## Claims

1. A process for producing a porous article comprising the following steps:
- preparing a ceramic slip in a liquid using a calcium phosphate powder and a deflocculating agent,
- modifying surface activity of the slip;
- homogenizing the slip by mixing it in a closed container containing grinding means and a predetermined volume of air or of an inert gas so as to make the slip porous,
- casting the porous slip thus obtained in a microporous mould,
- drying the porous slip cast in the microporous mould so as to obtain the porous article,
- sintering the so obtained porous article.

2. The process for the production of a porous article according to claim 1, wherein the volume of air or inert gas is in the range between 35% and 70% of the total volume of the slip plus the volume of air or inert gas itself.

3. The process for the production of a porous article according to claim 1 or 2, **characterised in that** the step of modifying the surface activity of the slip is achieved by adding a surfactant.

4. The process for the production of a porous article according to any of the foregoing claims, wherein the step of modifying the surface activity of the slip is achieved by adding one or more anionic surfactants selected from the group of alkyl sulphates, sodium salts and ammonium salts.

5. The process for the production of a porous article according to any of the foregoing claims, **characterised in that** the step of modifying the surface activity of the slip is achieved by mixing at low speed.

6. The process for the production of a porous article according to any of the foregoing claims, wherein the step of modifying the surface activity of the slip is achieved by placing the products used to make the slip in a container (2) rotating at a speed of between 10 and 20 rpm for a time of between 2 and 20 hours.

7. The process for the production of a porous article according to any of the foregoing claims, **characterised in that** the step of homogenizing the ceramic slip includes the use of hard and inert grinding means, in a ratio of between 0.75:1 and 1.5:1 relative to the quantity of slip.

8. The process for the production of a porous article according to any of the claims from 1 to 7, **characterised in that** the step of homogenizing the ceramic slip includes the use of hard and inert grinding means, in a ratio of 1:1 relative to the quantity of slip.

9. The process for the production of a porous article according to claim 7 or 8, **characterised in that** the grinding means are made of alumina.

10. The process for the production of a porous article according to claim 7 or 8, **characterised in that** the step of homogenizing the ceramic slip is achieved by mixing at a low speed of rotation of the closed container (2) in which the products used to make the slip are held.

11. The process for the production of a porous article according to claim 7 or 8, **characterised in that** the step of homogenizing the ceramic slip is achieved by mixing at a speed of rotation in the range between 40 and 80 rpm of the container (2) in which the products used to make the slip are held.

12. The process for the production of a porous article according to claim 7 or 8, **characterised in that** the step of homogenizing the ceramic slip is carried out for a time in the range between 2 and 20 hours.

13. The process for the production of a porous article according to any of the foregoing claims, **characterised in that** the step of casting the slip is achieved using a microporous paper mould.

14. The process for the production of a porous article according to claim 13, **characterised in that** the paper used to make the mould has a porosity in the range between 17 and 25 µm.

15. The process for the production of a porous article according to any of the foregoing claims, **characterised in that** the step of preparing the slip is achieved using calcium phosphate powders selected from a powder in the group consisting of hydroxyapatite (HA), Beta-Tricalcium Phosphate (β-TCP), Alpha-Tricalcium phosphate (α-TCP), carbonated hydroxyapatite (CHA) and hydroxyapatite with magnesium (HA-Mg).

16. The process for the production of a porous article according to any of the claims from 1 to 14, **characterised in that** the step of preparing the slip is achieved using a biphasic calcium phosphate powder selected from a biphasic powder in the group consisting of hydroxyapatite / Beta-Tricalcium Phosphate (HA / β-TCP) , hydroxyapatite / Alpha-Tricalcium Phosphate (HA/α-TCP) and Beta-Tricalcium Phosphate / Alpha-Tricalcium Phosphate (P-TCP/ α-TCP).

17. The process for the production of a porous article according to any of the foregoing claims, **characterised in that** the step of preparing the slip is achieved using a calcium phosphate powder with a surface area in the range between 2 and 8 m²/g.

18. The process for the production of a porous article according to any of the foregoing claims, **characterised in that** the step of preparing the slip is achieved using a deflocculating agent selected from alkali-free polyelectrolytes.

19. The process for the production of a porous article according to any of the foregoing claims, **characterised in that** the step of drying the slip takes place at room temperature for a time in the range between 24 and 48 hours.

20. The process for the production of a porous article according to any of the foregoing claims, **characterised in that** the step of drying the slip takes place with the aid of microwaves for a time in the range between 5 and 24 hours.

21. The process for the production of a porous article according to any of the foregoing claims, **characterised in that** the sintering step takes place at a temperature in the range between 400 and 1400 degrees Centigrade.

22. The process for the production of a porous article according to any of the foregoing claims, **characterised in that** the sintering step takes place at a temperature of 1200 degrees Centigrade.

23. The process for the production of a porous article according to any of the foregoing claims, **characterised in that** the sintering step takes place in a flow of carbon dioxide (CO₂).

## Patentansprüche

1. Verfahren zur Herstellung eines porösen Körpers, umfassend die folgenden Phasen:
- Vorbereiten eines keramischen Schlickers in einer Flüssigkeit mithilfe eines Calciumphosphatpulvers und eines Entflockungsmittels,
- Ändern der Oberflächenaktivität des Schlickers;
- Homogenisieren des Schlickers durch seine Vermischung in einem abgeschlossenen Behälter, der Zerkleinerungsmittel sowie eine vorgegebene Menge an Luft oder eines Inertgases enthält, sodass der Schlicker porös gemacht wird,
- Gießen des so erhaltenen porösen Schlickers in eine mikroporöse Gießform,
- Trocknen des in die mikroporöse Gießform gegossenen Schlickers, sodass der poröse Körper erhalten wird,
- Sintern des so erhaltenen porösen Körpers.

2. Verfahren zur Herstellung eines porösen Körpers nach Anspruch 1, wobei das Volumen an Luft oder Inertgas zwischen 35 und 70 % des Gesamtvolumens des Schlickers plus das Volumen der Luft oder des Inertgases beträgt.

3. Verfahren zur Herstellung eines porösen Körpers nach Anspruch 1 oder 2, **dadurch gekennzeichnet, dass** die Phase des Änderns der Oberflächenaktivität des Schlickers durch die Zugabe eines Oberflächenbehandlungsmittels erzielt wird.

4. Verfahren zur Herstellung eines porösen Körpers nach irgendeinem der vorhergehenden Ansprüche, wobei die Phase des Änderns der Oberflächenaktivität des Schlickers durch die Zugabe von einem oder mehreren anionischen Oberflächenbehandlungsmitteln erzielt wird, ausgewählt aus der Gruppe der Alkylsulfate, Natriumsalze und Ammoniumsalze.

5. Verfahren zur Herstellung eines porösen Körpers nach irgendeinem der vorhergehenden Ansprüche, **dadurch gekennzeichnet, dass** die Phase des Änderns der Oberflächenaktivität durch Vermischen bei niedriger Geschwindigkeit erzielt wird.

6. Verfahren zur Herstellung eines porösen Körpers nach irgendeinem der vorhergehenden Ansprüche, wobei die Phase des Änderns der Oberflächenaktivität des Schlickers **dadurch** erzielt wird, dass die zur Herstellung des Schlickers verwendeten Produkte in einen Behälter (2) gegeben werden, der sich für 2 bis 20 Stunden bei einer Drehzahl von 10 bis 20 Upm dreht.

7. Verfahren zur Herstellung eines porösen Körpers nach irgendeinem der vorhergehenden Ansprüche, **dadurch gekennzeichnet, dass** die Phase des Homogenisierens des keramischen Schlickers die Verwendung von harten und inerten Zerkleinerungsmitteln in einem Verhältnis von 0,75:1 bis 1,5:1 zur Schlickermenge beinhaltet.

8. Verfahren zur Herstellung eines porösen Körpers nach irgendeinem der vorhergehenden Ansprüche von 1 bis 7, **dadurch gekennzeichnet, dass** die Phase des Homogenisierens des keramischen Schlickers die Verwendung von harten und inerten Zerkleinerungsmitteln in einem Verhältnis von 1:1 zur Schlickermenge beinhaltet.

9. Verfahren zur Herstellung eines porösen Körpers nach Anspruch 7 oder 8, **dadurch gekennzeichnet, dass** die Zerkleinerungsmittel aus Aluminiumoxid bestehen.

10. Verfahren zur Herstellung eines porösen Körpers nach Anspruch 7 oder 8, **dadurch gekennzeichnet, dass** die Phase des Homogenisierens des keramischen Schlickers durch das Vermischen des abgeschlossenen Behälters (2), in dem die zur Herstellung des Schlickers verwendeten Produkte enthalten sind, bei geringer Drehzahl erzielt wird.

11. Verfahren zur Herstellung eines porösen Körpers nach Anspruch 7 oder 8, **dadurch gekennzeichnet, dass** die Phase des Homogenisierens des keramischen Schlickers durch das Vermischen des abgeschlossenen Behälters (2), in dem die zur Herstellung des Schlickers verwendeten Produkte enthalten sind, bei einer Drehzahl von 40 bis 80 Upm erzielt wird.

12. Verfahren zur Herstellung eines porösen Körpers nach Anspruch 7 oder 8, **dadurch gekennzeichnet, dass** die Phase des Homogenisierens des keramischen Schlickers für einen Zeitraum von 2 bis 20 Stunden durchgeführt wird.

13. Verfahren zur Herstellung eines porösen Körpers nach irgendeinem der vorhergehenden Ansprüche, **dadurch gekennzeichnet, dass** die Phase des Gießens des Schlickers anhand der Verwendung einer mikroporösen Papiergießform erzielt wird.

14. Verfahren zur Herstellung eines porösen Körpers nach Anspruch 13, **dadurch gekennzeichnet, dass** das zur Herstellung der Form verwendete Papier eine Porosität von 17 bis 25 µm aufweist.

15. Verfahren zur Herstellung eines porösen Körpers nach irgendeinem der vorhergehenden Ansprüche, **dadurch gekennzeichnet, dass** die Phase des Vorbereitens des Schlickers durch die Verwendung von Calciumphosphatpulvern erzielt wird, ausgewählt aus einem Pulver in der Gruppe, bestehend aus Hydroxyapatit (HA), Beta-Tricalciumphosphat (β-TCP), Alpha-Tricalciumphosphat (α-TCP), carboniertem Hydroxyapatit (CHA) und Hydroxyapatit mit Magnesium (HA-Mg).

16. Verfahren zur Herstellung eines porösen Körpers nach irgendeinem der vorhergehenden Ansprüche 1 bis 14, **dadurch gekennzeichnet, dass** die Phase des Vorbereitens des Schlickers durch die Verwendung eines biphasischen Calciumphosphatpulvers erzielt wird, ausgewählt aus einem biphasischen Pulver in der Gruppe, bestehend aus Hydroxyapatit/Beta-Tricalciumphosphat (HA / β-TCP), Hydroxyapatit/Alpha-Tricalciumphosphat (HA/α-TCP) und Beta-Tricalciumphospat/Alpha-Tricalciumphosphat (β-TCP/ α-TCP).

17. Verfahren zur Herstellung eines porösen Körpers nach irgendeinen der vorhergehenden Ansprüche, **dadurch gekennzeichnet, dass** die Phase des Vorbereitens des Schlickers durch die Verwendung eines Calciumphosphatpulvers mit einer Oberfläche zwischen 2 und 8 m²/g erzielt wird.

18. Verfahren zur Herstellung eines porösen Körpers nach irgendeinem der vorhergehenden Ansprüche, **dadurch gekennzeichnet, dass** die Phase des Vorbereitens des Schlickers durch die Verwendung eines Entflockungsmittels, ausgewählt aus alkalifreien Polyelektrolyten, erzielt wird.

19. Verfahren zur Herstellung eines porösen Körpers nach irgendeinem der vorhergehenden Ansprüche, **dadurch gekennzeichnet, dass** die Phase des Trocknens des Schlickers bei Raumtemperatur für einen Zeitraum von 24 bis 48 Stunden erfolgt.

20. Verfahren zur Herstellung eines porösen Körpers nach irgendeinem der vorhergehenden Ansprüche, **dadurch gekennzeichnet, dass** die Phase des Trocknens des Schlickers mithilfe von Mikrowellen für einen Zeitraum von 5 bis 24 Stunden erfolgt.

21. Verfahren zur Herstellung eines porösen Körpers nach irgendeinem der vorhergehenden Ansprüche, **dadurch gekennzeichnet, dass** die Phase des Sinterns bei einer Temperatur von 400 bis 1400 Grad Celsius stattfindet.

22. Verfahren zur Herstellung eines porösen Körpers nach irgendeinem der vorhergehenden Ansprüche, **dadurch gekennzeichnet, dass** die Phase des Sinterns bei einer Temperatur von 1200 Grad Celsius stattfindet.

23. Verfahren zur Herstellung eines porösen Körpers nach irgendeinem der vorhergehenden Ansprüche, **dadurch gekennzeichnet, dass** die Phase des Sinterns in einem Fluss von Kohlendioxid (CO₂) stattfindet.

## Revendications

1. Procédé pour fabriquer un article poreux, comprenant les phases suivantes :
- préparation d'une barbotine céramique dans un liquide avec une poudre de phosphate de calcium et un défloculant,
- modification de l'activité de surface de la barbotine,
- uniformisation de la barbotine via son mélange dans un récipient contenant des moyens de broyage et un volume prédéterminé d'air ou un gaz inerte pour rendre la barbotine poreuse,
- coulage de la barbotine poreuse obtenue dans un moule microporeux,
- séchage de la barbotine poreuse coulée dans le moule microporeux pour obtenir l'article poreux,
- frittage de l'article poreux ainsi obtenu.

2. Procédé pour fabriquer un article poreux selon la revendication 1, dans lequel le volume d'air ou de gaz inerte représente de 35% à 70% du volume total de la barbotine, plus le volume d'air ou de gaz inerte en lui-même.

3. Procédé pour fabriquer un article poreux selon les revendications 1 ou 2, **caractérisé en ce que** la phase de modification de l'activité de surface de la barbotine est obtenue par l'ajout d'un agent de surface.

4. Procédé pour fabriquer un article poreux selon l'une quelconque des revendications précédentes, dans lequel la phase de modification de l'activité de surface de la barbotine est obtenue par l'ajout d'un ou de plusieurs agents de surface anioniques sélectionnés dans le groupe des sulfates d'alkyle, des sels de sodium et des sels d'ammonium.

5. Procédé pour fabriquer un article poreux selon l'une quelconque des revendications précédentes, **caractérisé en ce que** la phase de modification de l'activité de surface de la barbotine est obtenue via un mélange à faible vitesse.

6. Procédé pour fabriquer un article poreux selon l'une quelconque des revendications précédentes, dans lequel la phase de modification de l'activité de surface de la barbotine est obtenue en plaçant les produits utilisés pour la barbotine dans un récipient (2) tournant à une vitesse comprise entre 10 et 20 tr/min pendant une durée comprise entre 2 et 20 heures.

7. Procédé pour fabriquer un article poreux selon l'une quelconque des revendications précédentes, **caractérisé en ce que** la phase d'uniformisation de la barbotine céramique comprend l'utilisation de moyens de broyage rigides et inertes, dans une proportion comprise entre 0,75:1 et 1,5:1 par rapport à la quantité de barbotine.

8. Procédé pour fabriquer un article poreux selon l'une des revendications 1 à 7, **caractérisé en ce que** la phase d'uniformisation de la barbotine céramique comprend l'utilisation de moyens de broyage rigides et inertes, dans une proportion de 1:1 par rapport à la quantité de barbotine.

9. Procédé pour fabriquer un article poreux selon les revendications 7 à 8, **caractérisé en ce que** les moyens de broyage sont en alumine.

10. Procédé pour fabriquer un article poreux selon les revendications 7 ou 8, **caractérisé en ce que** la phase d'uniformisation de la barbotine céramique est obtenue par mélange en faisant tourner à faible vitesse le récipient (2) fermé dans lequel se trouvent les produits utilisés pour préparer la barbotine.

11. Procédé pour fabriquer un article poreux selon les revendications 7 ou 8, **caractérisé en ce que** la phase d'uniformisation de la barbotine céramique est obtenue par mélange à une vitesse de rotation comprise entre 40 et 80 tr/min du récipient (2), dans lequel se trouvent les produits utilisés pour préparer la barbotine.

12. Procédé pour fabriquer un article poreux selon les revendications 7 ou 8, **caractérisé en ce que** la phase d'uniformisation de la barbotine céramique a une durée comprise entre 2 et 20 heures.

13. Procédé pour fabriquer un article poreux selon l'une quelconque des revendications précédentes, **caractérisé en ce que** la phase de coulage de la barbotine se fait dans un moule en papier microporeux.

14. Procédé pour fabriquer un article poreux selon la revendication 13, **caractérisé en ce que** le papier utilisé pour fabriquer le moule a une porosité comprise entre 17 et 25 µm.

15. Procédé pour fabriquer un article poreux selon l'une quelconque des revendications précédentes, **caractérisé en ce que** la phase de préparation de la barbotine se base sur des poudres de phosphate de calcium sélectionnées dans une poudre du groupe formé d'hydroxyapatite (HA), de phosphate tricalcique bêta (β-TCP), de phosphate tricalcique alpha (α-TCP), d'hydroxyapatite carbonée (CHA) et d'hydroxyapatite avec magnésium (HA-Mg).

16. Procédé pour fabriquer un article poreux selon l'une quelconque des revendications 1 à 14, **caractérisé en ce que** la phase de préparation de la barbotine se base sur une poudre de phosphate de calcium biphasique sélectionnée dans une poudre biphasique du groupe formé d'hydroxyapatite / phosphate tricalcique bêta (HA/β-TCP), d'hydroxyapatite / phosphate tricalcique alpha (HA/α-TCP) et de phosphate tricalcique bêta / phosphate tricalcique alpha (β-TCP/α-TCP).

17. Procédé pour fabriquer un article poreux selon l'une quelconque des revendications précédentes, **caractérisé en ce que** la phase de préparation de la barbotine se base sur une poudre de phosphate de calcium présentant une surface comprise entre 2 et 8 m²/g_{.}

18. Procédé pour fabriquer un article poreux selon l'une quelconque des revendications précédentes, **caractérisé en ce que** la phase de préparation de la barbotine se base sur l'utilisation d'un défloculant sélectionné dans les polyélectrolytes sans alcali.

19. Procédé pour fabriquer un article poreux selon l'une quelconque des revendications précédentes, **caractérisé en ce que** la phase de séchage se fait à température ambiante pendant une durée comprise entre 24 et 48 heures.

20. Procédé pour fabriquer un article poreux selon l'une quelconque des revendications précédentes, **caractérisé en ce que** la phase de séchage de la barbotine se fait à l'aide de micro-ondes pendant une durée comprise entre 5 et 24 heures.

21. Procédé pour fabriquer un article poreux selon l'une quelconque des revendications précédentes, **caractérisé en ce que** la phase de frittage se fait à une température comprise entre 400 et 1 400 degrés centigrades.

22. Procédé pour fabriquer un article poreux selon l'une quelconque des revendications précédentes, **caractérisé en ce que** la phase de frittage se fait à une température de 1 200 degrés centigrades.

23. Procédé pour fabriquer un article poreux selon l'une quelconque des revendications précédentes, **caractérisé en ce que** la phase de frittage se fait sous un flux de dioxyde de carbone (CO₂).
